# EUROPEAN PATENT APPLICATION

(11) **EP 3 763 358 A1**
(43) Date of publication of application: **13.01.2021**
(21) Application number: 20182834.0
(22) Date of filing: 29.06.2020
(51) Int. Cl.: A61K 9/10, A61K 47/36, A61K 33/10, A61K 31/198, A61K 31/7024, A61K 31/585, A61K 31/5513, A61K 31/4725, A61K 31/525, A61K 31/138

(54) **SUSPENDING VEHICLE FORMULATION**

(30) Priority: 09.07.2019 GB 201909826
(71) Applicant: Lexon UK Holding Limited, Redditch, Worcestershire B98 0RE (GB)
(72) Inventor: Patel, Yogesh, Redditch, Worcestershire B98 0RE (GB); DiPaolo, Toni-Bianca, Birmingham, West Midlands B15 2TT (GB); Zhang, Zhibing, Birmingham, West Midlands B15 2TT (GB); Preece, Jon, Birmingham, West Midlands B15 2TT (GB)
(74) Representative: HGF

(57) **Abstract**

A liquid suspending vehicle comprising arrowroot and xanthan gum, wherein the total solids content is less than 4% w/v. The weight ratio of arrowroot to xanthan gum may be from 13:1 or 12:1 to 6:1.

## Description

### FIELD OF THE INVENTION

The invention relates to a suspending vehicle formulation. In particular, but not exclusively, the formulation may be used to suspend solid medicament dosage forms.

### BACKGROUND OF THE INVENTION

Suspensions are an important class of medication for patients. With an increasing elderly population comes a higher demand for medications in liquid form for oral delivery. As well as geriatric patients, infants and children, animals, and patients having an illness or taking medication that can cause dysphagia, require alternatives to solid dosage forms. Suspensions are often easier to administer and allow for the effective dispensing of a wide range of medicaments, including hydrophobic drugs. Suspensions can avoid the need for cosolvents and may offer resistance to degradation of drugs due to hydrolysis, oxidation, or microbial activity. Accordingly, suspensions provide a means to treat patients with a variety of medications but in alternative forms.

Liquid suspending agents are ideally resistant to changes imparted on them by the addition of chemical ingredients (*e.g.* medicaments) and can prevent precipitation and caking of the active component at the base of the suspension.

The main classifications of suspending agents are polysaccharides (*e.g.* starch, cellulose, alginates and xanthan gums), inorganic salts (*e.g.* bentonite and aluminium hydroxide) and synthetics (*e.g.* carbomers and polyvinylpyrrolidone iodine).

Whilst suspending agents are required to permanently form suspensions, they are often limited by several drawbacks. Ingredients can sediment and be difficult to resuspend. Stability problems and physical changes can lead to precipitation, coagulation and deactivation of the suspended agent. Many formulations are hyperosmotic, reducing the number of compatible compounded medications that can be formulated and resulting in undesirable side effects such as bulk laxative effects. Current commercially available products have limited success in providing a sufficient shelf-life for water sensitive therapeutic agents in aqueous formulations.

US8021682 describes a palatable suspending vehicle for pharmaceutical ingredients. However, these formulations contain modified starches and starches obtained from a variety of sources, such as grains which include varieties of wheat, meaning these suspensions cannot be used by those patients with medical conditions such as celiac disease.

It would be advantageous to provide a vehicle capable of providing water-stable pharmaceutical compositions and liquid dosage forms derived therefrom employing therapeutic agents that minimise the effect of storage on the therapeutic agents and/or their mode of action. This is especially true in regard to water sensitive therapeutic agents, where hydrolysis may lead to the production of by-products that reduce overall efficacy and/or increase side effects, and in the case of pharmaceutical specials. It would be useful to provide a suspending agent which is able to be administered to those that have intolerance to gluten.

Pharmaceutical specials are a category of unlicensed medicinal products that are manufactured or procured specifically for individual patients. Specials are bespoke and prepared to meet a prescription ordered for the specific clinical needs of an individual that cannot be met by a licensed product. Specials are made up of a huge array of different formulations, from creams and liquids to solutions for intravenous delivery and injectables. They can be an unusual formulation, concentration or strength and may necessitate the need to be free from certain additives, preservatives or drug excipients. Therefore, a liquid formulation that can be specifically tailored for an individual, is capable of suspending solid medicament dosages for oral delivery, and that possesses enhanced stability, would be extremely advantageous.

### STATEMENTS OF INVENTION

An aspect of the present invention provides a liquid suspending vehicle comprising a liquid, arrowroot and a thickening agent, for example xanthan gum, wherein the total solids content is less than 4% w/v.

Arrowroot is both natural and gluten free. From our experiments we have found that the arrowroot based suspending agent need not include a surfactant, defoaming agent (or antifoaming agent) and/or a co-solvent.

The invention comprises arrowroot as a suspending agent. Where arrowroot may be 'true' Indian or West Indian arrowroot (*Maranta arundinacea*) as well as other species of *Maranta,* such as *Maranta ramosissima, Maranta allouya, Maranta nobilis, Maranta Indica,* along with Tous-les-mois, or Tulema arrowroot (Canna coccinea), Brazilian arrowroot (*Manihot utilissima* or *Manihot palmate*), Portland arrowroot derived from tubers of cuckoopint (*Arum macculatum*), Potato Farina or British arrowroot, Taro (*Colocasia esculenta*) or Nduma, Tahitian arrowroot (*Tacca oceanica*), East Indian arrowroot (*Curcuma augustifolia*), Florida arrowroot (*Zamia integrifolia*), tapioca from cassava (*Manihot esculenta*), Polynesian arrowroot or pia (*Tacca leontopetaloides*), Tacca or Otaheite arrowroot from the pia plant (*Pia pinnatifida*) and Japanese arrowroot (*Pueraria lobata*), also called kudzu.

Arrowroot starch from the *Maranta arundinacea* plant is considered a herb whereas, tapioca is obtained from the cassava root. Being derived from the rhizomes (rootstock), this starch containing formulation is gluten free, making it accessible to a larger number of consumers. This overcomes a potential issue arising with other starches, including maize and corn starch, where there are questions as to whether they comprise gluten. It also has a lower carbohydrate content than maize, corn starch and other modified starches. In addition, the arrowroot formulation has a neutral taste thus improved palatability. We have found that the formulation is capable of dissolving the film of some otherwise problematic tablets.

The invention comprises combining arrowroot with xanthan (corn sugar) gum. Xanthans, known commercially as xanthan gums (for example Keltrol®, Satiaxane™ and Verxan™) are common additives in the food industry.

Substitutes for the xanthan gums include hydrocolloids: agar (or agar-agar), grewia gum, guar gum, gum arabic, locust (carob) bean gum, gum tragacanth, konjac-glucomannan and carrageenan.

The liquid is preferably water.

We believe that xanthan gum enhances stability, for example freeze-thaw and/or pH stability. We believe that xanthan gum inhibits (e.g. prohibits) starch degradation and/or syneresis.

We have surprisingly found that the suspending vehicle allows for the dispensing of a wide range of medicaments, including hydrophobic drugs, and can be tailored for each individual. In addition, the formulation may incorporate a wide range and combination of functional ingredients, meaning many variations and modifications may be made.

This invention relates to a liquid suspending vehicle with improved stability and freeze-thaw properties. The suspension comprises an active ingredient and is suitable for use in a variety of industries, including, but not limited to medical and veterinary.

In an embodiment of the invention, the liquid suspending vehicle is for use as a delivery system, in particular but not exclusively, as an oral delivery system, for active ingredients. These active ingredients may be one or more of; medicaments, rehydration beverages, vitamins and/or supplements for nourishment and/or sustenance. The formulation is also suitable for ingestion by both animals and humans.

In particular, but not exclusively, the liquid suspending vehicle may be for use as a pharmaceutical special. Here the active ingredient is a therapeutic agent which can be suspended, dissolved or dispersed and directed to methods of prophylaxis and/or for diagnosis, cure, mitigation, treatment, or to prevent disease or symptoms thereof. The suspension allows for effective dispensing of a wide range of medications, including hydrophobic drugs and administration of extemporaneous preparations of oral, aqueous and insoluble dosage forms. The invention can also be specifically tailored to an individual, having an unusual formulation, concentration or strength or being free from certain additives, preservatives or drug excipients as required.

Advantageously, the liquid suspending vehicle is easier to administer than the alternate solid dosage forms, is a water-stable pharmaceutical composition and can offer resistance to degradation of drugs. We have found that in our experiments a co-solvent need not be present.

In embodiments of the invention the liquid suspending vehicle may be one or more of; shear-thinning, thixotropic and resistant to changes imparted on them by addition of active ingredients (e.g. medicaments). Advantageously, the formulation of the liquid suspending vehicle may also prevent precipitation and caking of the active component.

Advantageously, fewer additives and preservatives are required when using arrowroot as a suspending agent, in contrast to those currently available. This allows for generalised formulations to be produced in a more time-effective manner and at a lower cost. Tailored products, such as pharmaceutical specials, can be manufactured on a much shorter timescale and in line with demand, limiting wastage.

Advantageously, we have surprisingly found that the addition of xanthan gum to arrowroot at least partially prevents starch degradation and syneresis from occurring. We believe, although we do not wish or intend to be bound by any particular theory, that these effects occur through hydrogen bonding of the starch fragments. This combination of suspending agents allows properties such as the viscosity, appearance and flowability of the formulation to remain the same after it has been frozen and subsequently thawed. In effect, this means that the formulation does not degrade and hence the stability and therefore shelf-life of the formulation (both with and without the active ingredient) may be enhanced by this way of storage.

We believe that acid hydrolysis reduces the size of the amylose and amylopectin molecules on the arrowroot, leading to a lower viscosity. Viscosities were shown to be significantly increased with increasing xanthan gum concentrations, attributed to the double helix structure of xanthan gum reducing starch retrogradation by interacting with starch granules.

It is also thought that xanthan gum may prevent shear disruption, hydrogen bonding to starch when the shear rate is decreased.

In an embodiment of the invention the arrowroot content (w/v%) is greater than the xanthan gum content (w/v%).

In another embodiment of the invention, the arrowroot content may be less than 4% w/v, say in the range 2.0-3.9% w/v. We believe that an arrowroot concentration of 2.0-4.0 w/v% (or 2.0 to less than 4% w/v) provides a desired storage and viscosity profile.

In embodiments, the arrowroot content may be between any one of 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8% w/v, to any one of 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1% w/v.

Preferably, the arrowroot content may be in the range 3.0-3.5% w/v, for example between 3.1 to 3.4% w/v, or between 3.2 to 3.3% w/v. In embodiments, the arrowroot content may be 3.0% w/v.

In an embodiment of the invention the content of xanthan gum may be 0.5% w/v or less, say in the range 0.05-0.5% w/v. Preferably, xanthan gum may be in the range 0.25-0.5% w/v.

In a further embodiment of the invention the ratio of arrowroot to xanthan gum may be altered. The ratio of arrowroot to xanthan gum may be 14:1, 13:1, 12:1, 11:1, 10:1. 9:1, 8:1, 7:1 or 6:1. Preferably, the ratio of arrowroot (w/v):xanthan gum (w/v) may be 12:1, where the ratio of arrowroot to xanthan gum may be based on 3% w/v arrowroot, say 3% w/v arrowroot:0.25% w/v xanthan gum. Alternatively, it may comprise 3% w/v arrowroot:0.5% w/v xanthan gum or 3.25% w/v arrowroot:0.25% w/v xanthan gum.

In embodiments, the pH of the final liquid suspending vehicle composition may be in the range 4.0-4.5. For example, the pH of the final liquid suspending vehicle composition may be between any one of 4.0, 4.1, 4.2, 4.3, or 4.4 to any one of 4.5, 4.4, 4.3, 4.2 or 4.1.

The liquid suspending vehicle may further comprise one or more of: preservative, buffer, flavouring and/or sweetener. With physiological compatibility being maintained by controlling osmolality, pH and sweetener content.

Examples of suitable buffers include citric acid, sodium citrate and/or sodium phosphate.

Examples of suitable sweeteners include sucralose, sorbitol, stevia, monk fruit sweetener and sucrose.

Examples of suitable preservatives include potassium sorbate, sodium benzoate, methylparaben and propylparaben.

In an embodiment of the invention the preservative may also act as an anti-microbial and/or anti-oxidising agent.

A general formulation for an embodiment of a liquid suspending vehicle of the invention comprises the following:

**Table 1.**

| Ingredient | Range (percentage weight by volume relative to water) |
|---|---|
| Arrowroot starch | 3.0-3.5% |
| Xanthan gum | 0.25-0.50% |
| Preservative | 0.10% |
| Sweetener | <1% |
| Buffer | 0.33% |
| Flavouring | ∼1-2% |

In an embodiment of the invention the pH may be in the range 4.0-4.5. For example, a pH of between any one of 4.0, 4.1, 4.2, 4.3, or 4.4 to any one of 4.5, 4.4, 4.3, 4.2 or 4.1. Whereas current formulations comprising arrowroot starch and xanthan gum teach away from acidic pH, with pH 7 the most effective at reducing syneresis and pH 3 being the least effective (Sae-Kang et. al., Polymers, 2006, 65, pp 371-380).

In a further embodiment an active ingredient may be added, wherein the concentration of the active ingredient may be in the range 0.5 - 30 w/v%, say 2 - 25 w/v%, for example 2-21% w/v. In particular, but not exclusively, the functional ingredient may be a medicine. It will also be appreciated by those skilled in the art that more than one functional ingredient may be added.

Examples of active ingredients include: co-careldopa, clonazepam, solifenacin, sucralfate, diazepam, methyldopa, bisprolol, acetazolamide, amitriptyline, pizotifen, mesalazine, sertraline, calcium carbonate, sodium valproate, clopidogrel, quinine sulphate, phenytoin, hydroxyzine, co-dydramol, metformin, clonidine, diclofen, bendroflumethiazide.

The optimal concentration of xanthan gum when in combination with 3.0% w/v arrowroot starch and in the presence of an active is 0.25% w/v, with 0.5% w/v xanthan gum being too viscous when adding certain actives.

In embodiments, the liquid suspending vehicle may comprise an arrowroot concentration of 2.0 to less than 4.0% w/v (e.g. between 3.0 to 3.5% w/v of arrowroot), the ratio of arrowroot to xanthan gum may be between 13:1 to 6:1, and the pH may be between 4.0-4.5. It has been surprisingly found that this combination of features provides a particularly advantageous formulation of the invention.

In embodiments, the liquid suspending vehicle may comprise an arrowroot concentration of 2.0 to less than 4.0% w/v (e.g. between 3.0 to 3.5% w/v of arrowroot), the xanthan gum concentration may be between 0.05 to 0.5% w/v (e.g. between 0.25 to 0.5% w/v), and the pH may be between 4.0-4.5. It has been surprisingly found that this combination of features provides a particularly advantageous formulation of the invention.

As employed throughout the disclosure of the present invention the following terms, unless otherwise indicated, should be understood to have the following meanings.

As used herein, the term "water-stable" refers to the ability of the active ingredient, in the presence of water, to retain substantially all the necessary therapeutic, chemical and/or physical properties and their mode of action that were associated with the active ingredient prior to contact with water.

As used herein, the term "liquid form for oral delivery" or "liquid dosage form" refers to a dosage from wherein the therapeutic agent is contained or suspended within an acceptable medium, which is preferably predominantly water.

As used herein, the term "actives", "active ingredient" or "functional ingredient" refers to an ingredient which may be a compound, component, constituent, additive or element which brings about a therapeutic, chemical or physical effect, or mode of action, which is suitable for use in one of a number of industries, including but not limited to medical and veterinary.

For the avoidance of doubt, the terms "may", "and/or" and any similar term as used herein should be interpreted as non-limiting such that any feature so-described need not be present.

### BRIEF DESCRIPTION OF THE FIGURES

The invention will now be described by way of example only and with reference to the accompanying drawings in which:
Figures 1A-1B are graphs of viscosity vs shear rate, displaying the effect of the different components on the formulation;
Figure 2 is a graph of viscosity vs shear rate for vehicles of the invention;
Figures 3A-3B are graphs of viscosity vs shear rate for vehicles of the invention after being subject to a freeze-thaw cycle;
Figures 4A-4B are graphs of viscosity vs shear rate for different sources of arrowroot;
Figure 5 is a graph of viscosity vs shear rate, comparing different formulation scales;
Figures 6A-6C are graphs of viscosity vs shear rate for various actives;
Figures 7A-7I are graphs of viscosity vs shear rate for various actives after being subject to a freeze-thaw cycle.

An exemplary method for forming the formulation is as follows:
i. Adding arrowroot starch and xanthan gum (where present) to water;
ii. Heating the mixture to an elevated temperature (e.g. to 70°C) at, for example 700 rpm;
iii. Cooling the mixture to room temperature;
iv. Adding a buffer solution to the room temperature mixture of Step (iii) at 700 rpm;
v. Adjusting the pH of the resulting formulation, as necessary, to the required pH.

Preferably Step (iii) is performed once, e.g. as soon as, the mixture reaches 70°C.

Additionally, if required, Step (iv) may comprise adding for example a preservative, a sweetener, a surfactant, an antifoaming agent and/or a flavouring.

In the below examples the following protocol is deployed to provide the formulation.
A 5 L jacket stirred vessel (FerMac 200 Bioreactor Fermenter, Electrolab biotech with 6 blade Rushton Blade Turbine (RBT) and 4 baffles) which also comprises a temperature control unit (FerMac 240 temperature control unit, Electrolab biotech, and a FerMac 310 stirrer control unit, Electrolab biotech) was used to create the formulation.
All ingredients are added *via* a funnel which is placed in one of the ports on the lid of the vessel. The port is then re-sealed using a metal cap once the ingredients have been added.
Arrowroot starch is added slowly into the water and stirred at 700 rpm, ensuring that the arrowroot is fully dispersed in the formulation before adding more, as arrowroot can tend to clump if too much is added at one time or if there is an insufficient mixing speed. If required, once all the arrowroot has been added, the same process is used to introduce the xanthan gum.
The mixture is heated until it reaches 70°C. Whilst continuing to stir the mixture at 700 rpm the mixture is then cooled by removing the heating jacket. Alternatively, the mixture may be cooled via the jacket on the vessel. Alternatively, an external cooling unit could be connected to the instrument.
The mixture is cooled to <40°C, preferably room temperature.
Preferably, the preservative, sweetener and flavouring are added to the mixture and stirred (*e.g.* for 5 minutes at 700 rpm) before the buffer is added and stirred (e.g. for a further 5 minutes at 700 rpm). Excess buffer may be added to adjust the pH of the resulting formulation to the required pH.

In each case where viscosity is measured the following protocol is deployed.
An AR-G2 rheometer (TA Instruments) with a 60 mm diameter 2° cone was used for rheometer measurements. Each measurement required 3-5 ml of sample to be placed on the stationary, fixed peltier plate. The cone is then lowered to the set geometry gap (59 mm) with any residue or spillage from the side of the cone being removed. Conversely, more sample is added if gaps are present around the edges of the cone.
A pre-set procedure (shear rate ramp) is then started. Once a set temperature (20°C) is reached the sample undergoes pre-shearing at a shear rate of 10 s⁻¹ for 60 s to equilibrate the system. Following pre-shearing the sample undergoes shearing with shear rates ramped from 0.01 to 1000 s⁻¹ with twenty points per decade. Each sample being held for 20 s at each of the shear rate values. During the measurements both the shear stress and the viscosity are measured in order to determine the behaviour of the fluids. After the final shear at 1000 s⁻¹ the shearing is stopped, and data is recorded.

In each case where the formulation undergoes one or more freeze-thaw cycle, viscosity results after the freeze-thaw cycle are compared to the measurement taken prior to freezing. Appearance is determined visually, comparing the samples before and after freezing, and is dependent on: colour, consistency (i.e. no clumps of arrowroot), pourability, any separation (syneresis) of the components of the formulation and/or any sedimentation of particles.

### EXAMPLES

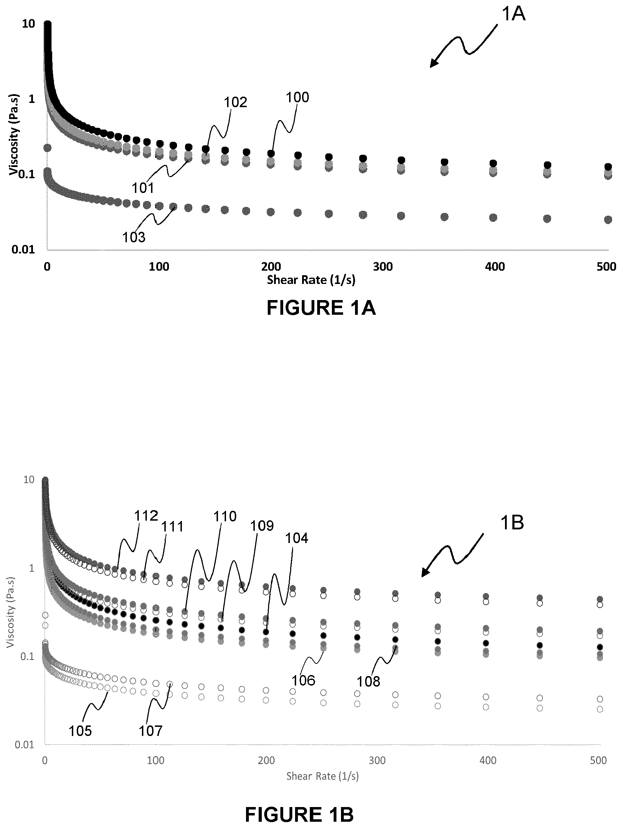

Method development for a formulation is presented in Figures 1A-1B.

### Example 1

Referring now to Figure 1A and Table 2, the effect of the different components on a formulation, in comparison to a commercial vehicle, are displayed. The rheology of the formulation comprising only arrowroot (4% w/v) is comparable to that of the commercial vehicle. The addition of preservative and sweetener to the formulation appears to have no impact on rheology and therefore no effect on stability. However, the addition of buffer (citric acid and sodium citrate, pH 4.5) appears to have a dramatic impact on the behaviour of the formulation, with the viscosity of the formulation being significantly reduced (approximately five times). The pH of the formulation is therefore viscosity limiting.

**Table 2. A formulation comprising different compositions and the curves that they are represented by in Figure 1A.**

| Formulation | Curve |
|---|---|
| Commercial Vehicle | 100 |
| Arrowroot alone | 101 |
| Arrowroot and preservatives | 102 |
| Arrowroot and buffer | 103 |

### Examples 2-4

Referring now to Figure 1B and Table 3, the addition of xanthan gum to a formulation comprising 4% w/v arrowroot appears to have a surprising effect on the viscosity of the formulation. Irrespective of whether buffer is present, the addition of xanthan gum is shown to stabilise the viscosity of the formulation, even at high solid contents. This is in stark contrast to the formulations (for example in Example 1) comprising arrowroot alone, where a significant change in viscosity is observed. Addition of xanthan gum therefore changes the formulation from being pH/viscosity limiting, to a stable formulation independent of buffer. Without wishing to be bound by theory, we believe that starch is sensitive to acid hydrolysis and degrades into fragments. These starch fragments then bind to xanthan gum through hydrogen bonding and in turn this improves pH stability. This appears to be independent of the suspending agent concentration and/or final solid content.

These Examples were prepared during the development of the formulation of Example 5. These do not represent examples of the invention.

**Table 3. Formulations comprising different compositions and concentrations, in the absence and presence of buffer, and the curves that they are represented by in Figure 1B.**

| Formulation | Measurement with buffer | Measurement without buffer |
|---|---|---|
| Commercial Vehicle | Curve 104 | - |
| 4% w/v arrowroot | Curve 105 | Curve 106 |
| 4.5% w/v arrowroot | Curve 107 | Curve 108 |
| 4.5% w/v arrowroot and 0.25% w/v xanthan gum | Curve 109 | Curve 110 |
| 4.5% w/v arrowroot and 0.5% w/v xanthan gum | Curve 111 | Curve 112 |

This clearly shows that the addition of a thickening agent (e.g. xanthan gum) mitigates the thinning effect of buffer addition.

### Example 5

A formulation according to the following composition was prepared.

| Ingredient | Range (percentage weight by volume relative to water) |
|---|---|
| Arrowroot starch | 3% |
| Xanthan gum | 0.25% |
| Potassium sorbate | 0.10% |
| Sucralose | 0.075% |
| Citric acid | 0.12% |
| Sodium citrate | 0.11% |

The formulation was prepared on a 5 L scale wherein the arrowroot starch (obtained from a commercial supplier, Bristol Botanicals Ltd.) and xanthan gum were dissolved in 3200 ml of water.

The formulation has the following features: it is thixotropic, comprises a flow index of 0.46 and/or a yield value of between 10-60 dynes/cm².

The formulation was prepared in the absence of a surfactant, defoaming agent and co-solvent.

### Example 5A

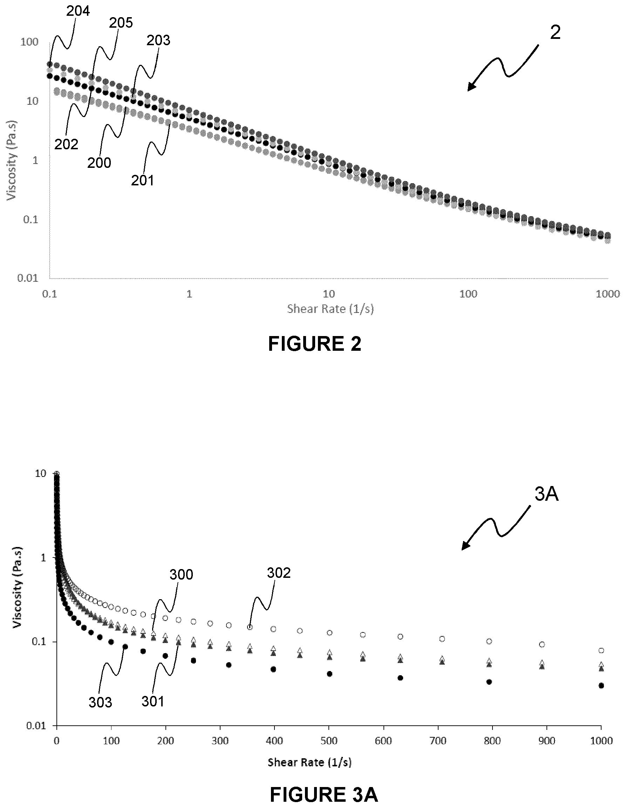

Referring now to Figure 2 and Table 4, the shelf-life stability of the formulation of Example 5 was tested at different time points. The formulations were stored under accelerated storage conditions (40°C with 75% relative humidity) in a stability chamber for the effective amount of days shown (1 week of accelerated storage corresponds to 28 days).

Rheology is used as a base point to determine stability. The formulation displayed no significant difference in rheology, up to 122 days testing.

**Table 4. The effective number of days the formulation was stored and the respective curve of Figure 2.**

| Effective Number of Days | Curve |
|---|---|
| 0 | 200 |
| 7 | 201 |
| 38* | 202 |
| 66 | 203 |
| 94 | 204 |
| 122 | 205 |

| | |
|---|---|
| *10 days after being formulated, the formulation was placed in the stability chamber for 1 week (∼28 days). | |

Viscosity is not only key in terms of storage and in creating a suspension but also for creating a desirable mouthfeel, a shear rate of between 20 and 50 s⁻¹ is considered optimal. In the optimal range there is little rheological change (Figure 2) which signifies that the formulation is stable over the test period (122 days).

### Example 5B

The freeze-thaw stability of the formulation of Example 5 was tested by analysing the rheological behaviour, viscosity and appearance. Samples from the same batch of formulation were transferred to 25 ml vials. The viscosity of the formulations was measured at 20°C, 1) as an initial basepoint and 2) after a first freeze-thaw cycle, where the freeze-thaw cycle involved the formulation being frozen at -15°C for at least 24 hours and subsequently completely thawed at room temperature for at least 4 hours.

Referring now to Figure 3A and Table 5, there is provided a comparison of the freeze-thaw stability of the formulation (as in Example 5) and a commercially available suspending agent (SyrSpend SF) after a single freeze-thaw cycle. The formulation displays little rheological change after being frozen for 48 hours and subsequently thawed. In contrast, a significant difference in viscosity is observed for the commercially available suspending agent. The formulation therefore shows enhanced freeze-thaw stability over a currently available product. This is apparent in the optimal 20-50 s⁻¹ shear rate range.

**Table 5. Curves representing the rheology measurements, in Figure 3A.**

| Suspending Vehicle | Initial Measurement | Measurement after a Freeze-thaw Cycle |
|---|---|---|
| Formulation | Curve 300 | Curve 301 |
| Commercial vehicle | Curve 302 | Curve 303 |

### Example 5C

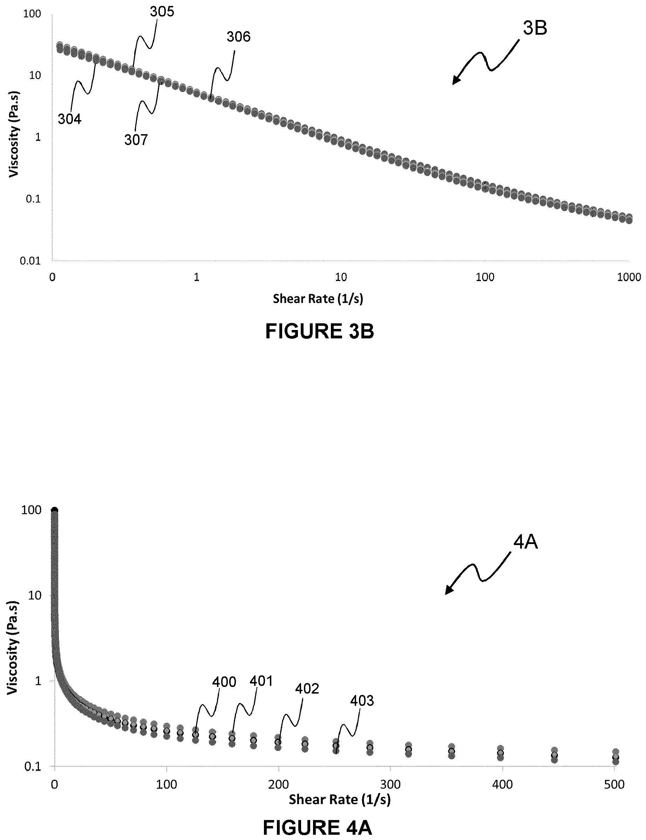

Referring now to Figure 3B and Table 6, there is provided the freeze-thaw stability measurements of the formulation of Example 5, tested over time. The rheological behaviour, viscosity and appearance of the formulation remain unchanged, as opposed to the properties of the prior art, where an increase in viscosity was observed (Figure 3A). This was particularly evident between the shear rates 20 to 50 s⁻¹, the optimal viscosity for oral suspensions. It demonstrates that the formulation is stable for up to 3 months.

**Table 6. The length of time the formulation was left before testing the rheology and the curves that represent these measurements in Figure 3B.**

| Time | Curve |
|---|---|
| Day 0 | 304 |
| 2 weeks | 305 |
| 1 month | 306 |
| 3 months | 307 |

### Examples 6-8

Referring now to Figure 4A and Table 7, to further exemplify the invention, the rheology of the formulation (as in Example 5) was measured wherein the formulations comprised arrowroot from different sources. Each of the formulations comprised an arrowroot content of 4.5% w/v and achieved a similar viscosity when compared to the commercially available suspending agent SyrSpend (Curve 400).

**Table 7. Formulations comprising different sources of arrowroot, in the absence and presence of xanthan gum, and the curves that they are represented by in Figures 4A-4B.**

| Arrowroot Source | Arrowroot Alone | Arrowroot and Xanthan Gum |
|---|---|---|
| Dr Oetker | Curve 401 | Curve 405 |
| Bristol Botanicals Ltd. | Curve 402 | Curve 406 |
| Ingredients UK arrowroot | Curve 403 | Curve 407 |

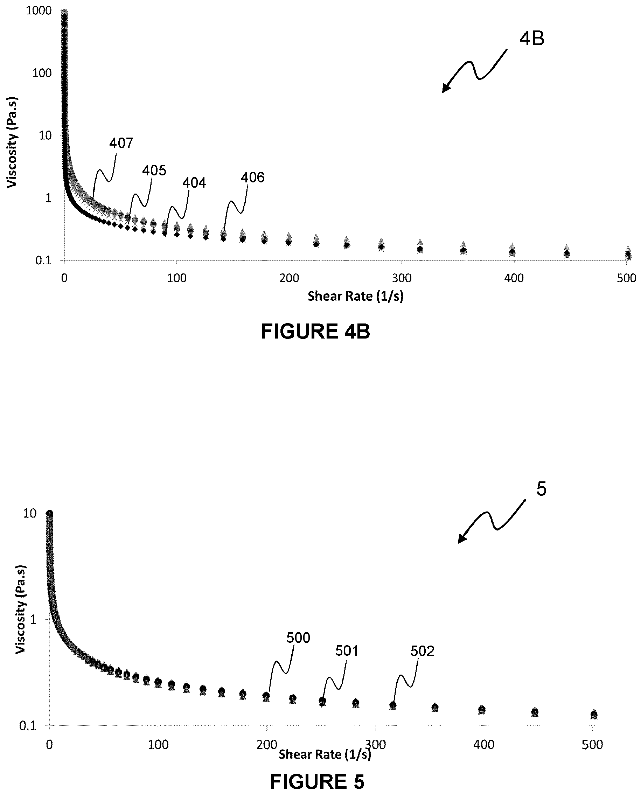

Figure 4B, as in Figure 4A, displays a comparison of viscosities for formulations as in Example 5 wherein the formulations comprised arrowroot from different sources. However, this time the formulations also comprised xanthan gum (0.25% w/v). Additionally, the arrowroot content was reduced to 4.25% w/v, in order that the overall solid content remained low at 4.5% w/v and to ensure that a good pourability was maintained.

Little difference was observed between the rheological measurements from the formulations comprising different sources of arrowroot, indicating that the rheological behaviour of the formulation is independent of the source of arrowroot used.

Referring to Figure 4B, the formulation comprising xanthan gum and different sources of arrowroot were compared to the commercially available suspending agent SyrSpend (Curve 404). The SyrSpend formulation displayed a slightly lower viscosity at lower shear rates (0-100 s⁻¹). Whilst the difference does not appear significant, we believe that the mouthfeel of the formulation and/or the stability of the suspension, (with higher viscosity being more desirable for better suspendability of actives) is preferable. We believe that this can be explained by the addition of xanthan gum to the formulations.

### Example 9

All processes disclosed in association with the present invention are contemplated to be practiced on any scale, but in particular lab scale (100 ml) and 5 L scale (Example 5). Referring now to Figure 5 and Table 8, there is provided a comparison of formulations (as in Example 5) made on different scales and in comparison to a model vehicle made on an industrial scale (for example made in 1000 L batches), which in this example is the commercial suspending agent SyrSpend. No difference in viscosity is observed on changing the shear rate, demonstrating that the formulation is both scalable and comparable to that of the model vehicle.

**Table 8. Formulations made on different scales and denoting the source of arrowroot they comprise and the curves that represent them in Figure 5.**

| Arrowroot Source, Commercial Supplier | Scale | Curve |
|---|---|---|
| Commercial Vehicle | 1000 L | 500 |
| Dr Oetker | 100 ml | 501 |
| Bristol Botanicals Ltd. | 5 L | 502 |

The subsequent examples comprise the formulation according to Example 5, but with the addition of various actives. The formulation has been shown to dissolve the film of some problematic tablets, for example methyldopa, mesalazine, metformin and/or diclofenac. Such actives usually require addition of a surfactant or co-solvent in order to dissolve the film.

### Examples 10-12

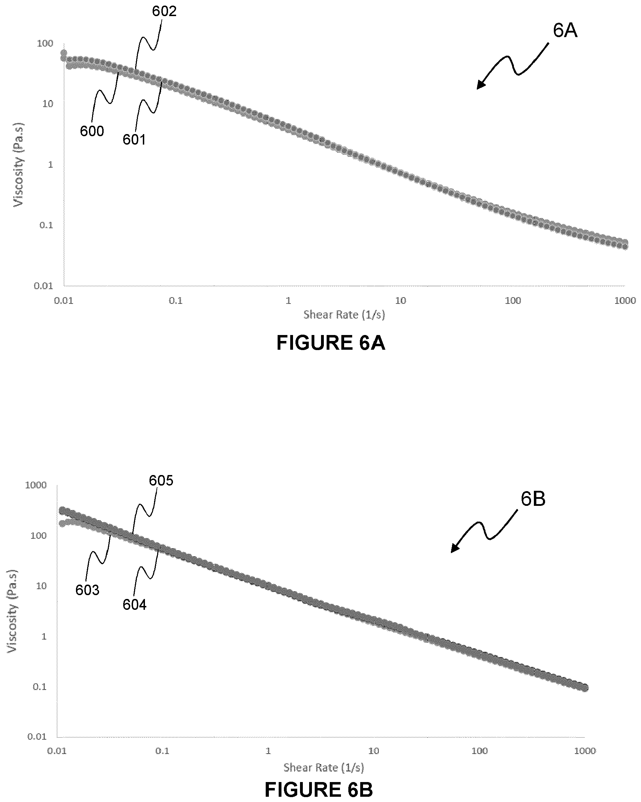

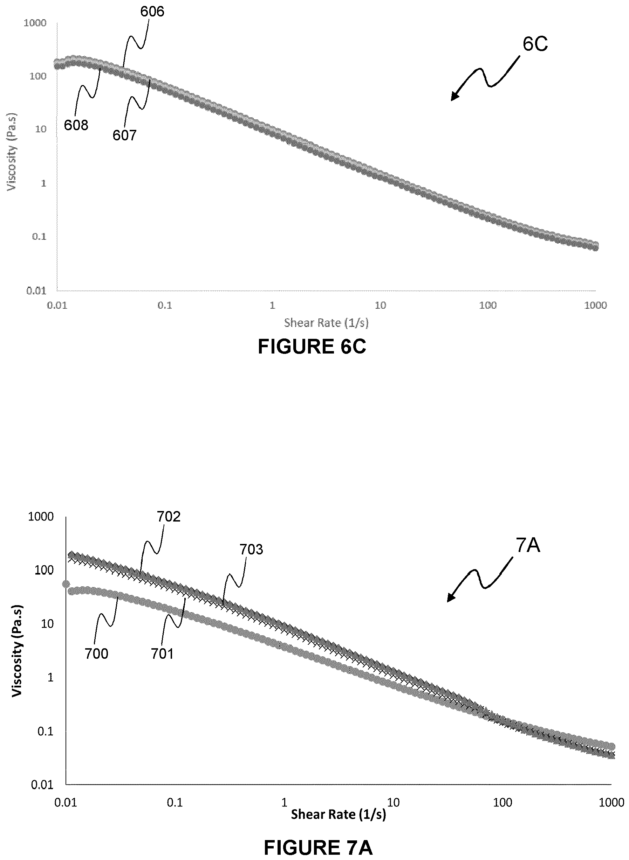

Referring now to Figures 6A-6C and Table 9, there is provided the shelf-life stability of the formulation in the presence of various actives. Rheology measurements were taken initially (day 0) and again after 7 days (freeze-thaw cycle 1) and 28 days, 1 week in a stability chamber (freeze-thaw cycle 2). As with the formulations comprising no active ingredient (Figure 2), there is little rheological change, meaning the formulation is stable at 28 days (∼1 month), after two freeze-thaw cycles, in the presence of various actives.

**Table 9. Details of curves from Figures 6A-6C representing rheology measurements, after being subject to freeze-thaw cycles, for formulations comprising various actives.**

| Figure | Medicament (Concentration) | Initial Measurement | Measurement after First Freeze-thaw Cycle | Measurement after Second Freeze-thaw Cycle |
|---|---|---|---|---|
| 6A | Co-careldopa (5% w/v) | Curve 600 | Curve 601 | Curve 602 |
| 6B | Sucralfate (13% w/v) | Curve 603 | Curve 604 | Curve 605 |
| 6C | Spironolactone (6% w/v) | Curve 606 | Curve 607 | Curve 608 |

### Examples 13-20

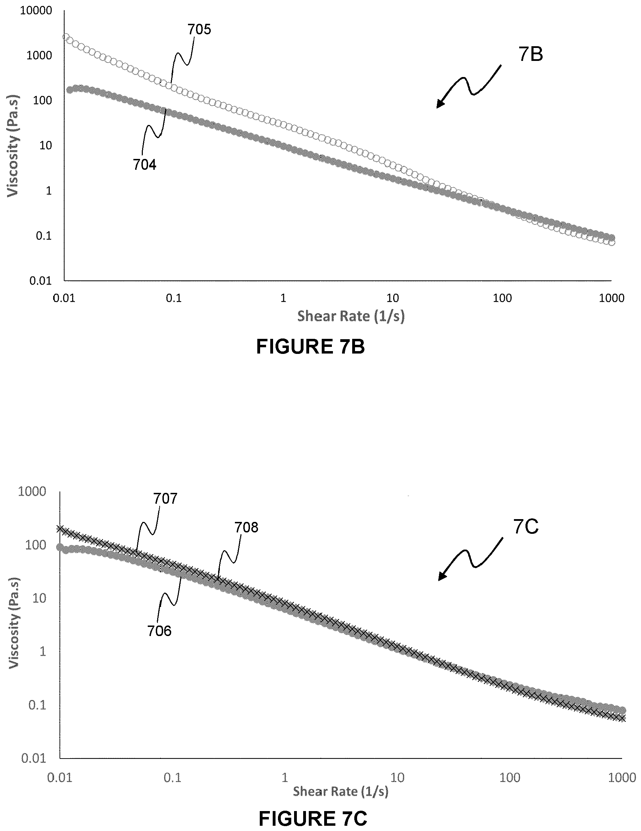

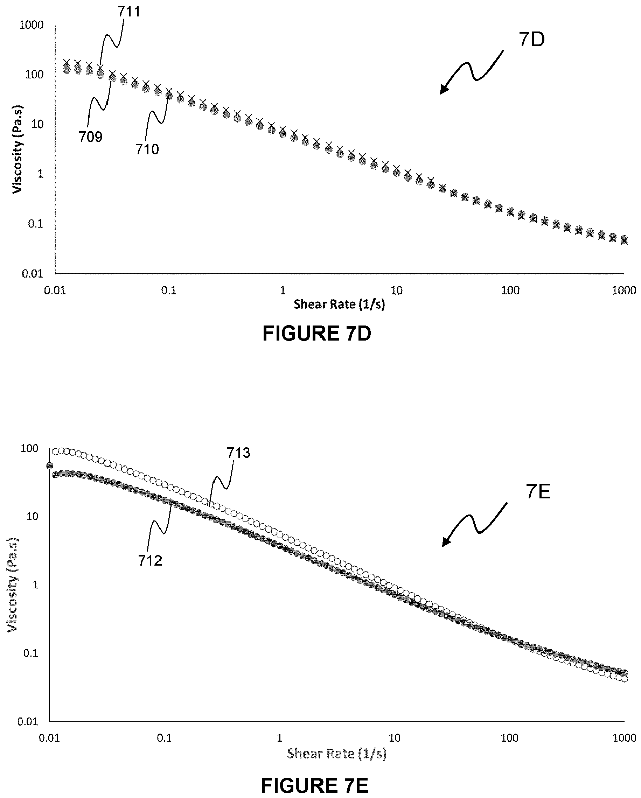

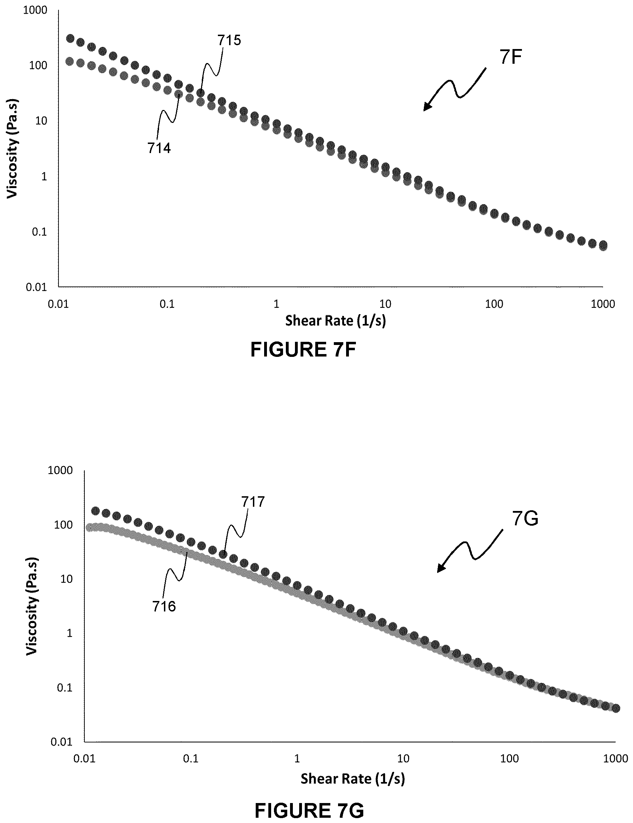

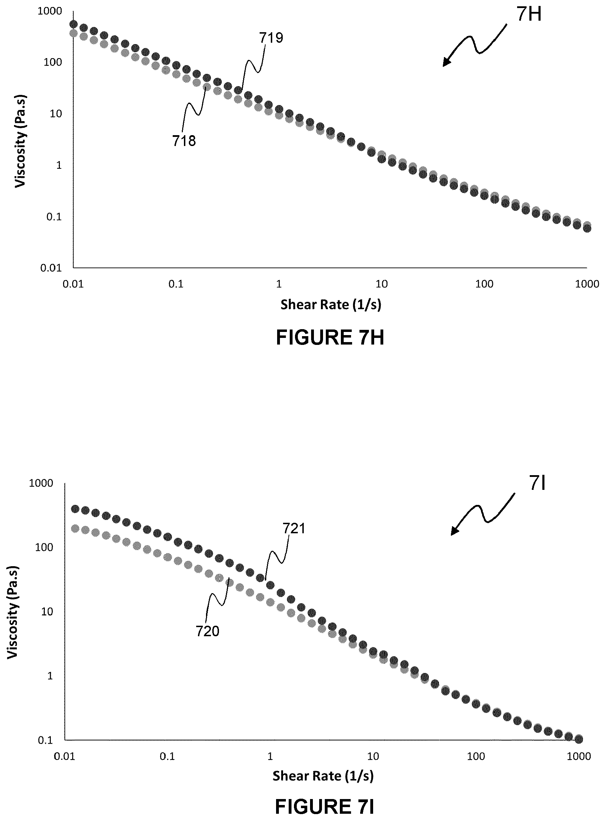

As observed with Figures 2A-2B, Figures 7A-7I display the freeze-thaw stability of the formulation but this time with various actives. Again, rheology measurements were taken initially and after being subject to freeze-thaw cycles. The addition of an active ingredient appears to have no impact on the freeze-thaw stability of the formulation, with little change in viscosity observed in the optimal 20-50 s⁻¹ shear rate range. This in effect means the starch formulation does not degrade and hence the stability of the formulation, both with and without the active ingredient, can be enhanced by this way of storage. Table 10 details the various actives tested and the freeze-thaw cycles that they were subject to.

**Table 10. Curves representing rheology measurements in Figures 7A-7I, for the formulation comprising various actives, after being subject to varying freeze-thaw cycles.**

| Figure | Medicament (Concentration) | Day 0 | 24 Hours | Day 7 | Day 14 | Day 28 | 4 Months |
|---|---|---|---|---|---|---|---|
| 7A | Co-careldopa (5% w/v) | Curve 700 | - | Curve 701 | Curve 702 | - | Curve 703 |
| 7B | Sucralfate (13% w/v) | Curve 704 | Curve 705 | - | - | - | - |
| 7C | Clonazepam (21% w/v) | Curve 706 | Curve 707 | - | - | Curve 708 | - |
| 7D | Solifenacin (3% w/v) | Curve 709 | Curve 710 | - | - | Curve 711 | - |
| 7E | Diazepam (9% w/v) | Curve 712 | Curve 713 | - | - | - | - |
| 7F | Methyldopa (7% w/v) | Curve 714 | Curve 715 | - | - | - | - |
| 7G | Bisprolol (4% w/v) | Curve 716 | Curve 717 | - | - | - | - |
| 7H | Riboflavin (2% w/v) | Curve 718 | Curve 719 | - | - | - | - |
| 7I | Calcium Carbonate (17% w/v) | Curve 720 | Curve 721 | - | - | - | - |

The actives in the above examples include commonly requested specials and/or problematic actives. For example, they may require co-solvents and/or surfactants to dissolve the film on the tablet or they may be bulky and/or viscous (for example methyldopa) making them difficult to prepare.

The formulations described in the above examples are shown to exhibit good viscosities both before and after the addition of an active ingredient, have a glossy shine and have a good pourability.

The vehicle is clear in colour in the absence of an active ingredient. The addition of an active may provide a coloured formulation. For example, diazepam provides a blue coloured formulation and methyldopa a pink formulation.

The formulations may be used in a variety of industries, including, but not limited to medical and veterinary, depending upon the active ingredient(s) selected.

It will also be appreciated by those skilled in the art that one or more functional ingredient(s) can be used in combination, unless such combinations are incompatible, and are therefore within the scope of the invention described herein.

## Claims

1. A liquid suspending vehicle comprising arrowroot and xanthan gum, wherein the total solids content is less than 4% w/v.

2. A liquid suspending vehicle of Claim 1, wherein the arrowroot is selected from 'true' Indian or West Indian arrowroot (Maranta arundinacea), Maranta ramosissima, Maranta allouya, Maranta nobilis, Maranta Indica, Tous-les-mois, Tulema arrowroot (Canna coccinea), Brazilian arrowroot (Manihot utilissima or Manihot palmate), Portland arrowroot (Arum macculatum), Potato Farina or British arrowroot, Taro (Colocasia esculenta) or Numda, Tahitian arrowroot (Tacca oceanica), East Indian arrowroot (Curcuma augustifolia), Florida arrowroot (Zamia integrifolia), tapioca from cassava (Manihot esculenta), Polynesian arrowroot, pia (Tacca leontopetaloides), Tacca, Otaheite arrowroot from pia plant (Pia pinnatifida) or Japanese arrowroot (Pueraria lobata).

3. A liquid suspending vehicle according to Claim 1 or 2, wherein the arrowroot content is greater than the xanthan gum content, for example wherein the weight ratio of arrowroot to xanthan gum is from 13:1 or 12:1 to 6:1.

4. A liquid suspending vehicle according to Claim 3, wherein the arrowroot content is less than 4% w/v, say in the range 2.0-3.9% w/v, for example the arrowroot content is in the range 3.0-3.5 % w/v.

5. A liquid suspending vehicle according to any preceding Claim, wherein the xanthan gum content is 0.5% w/v or less, say in the range 0.05-0.5.0% w/v, for example the xanthan gum content is in the range 0.25-0.5.0% w/v.

6. A liquid suspending vehicle according to any preceding Claim, wherein the pH is in the range pH 4.0-4.5.

7. A liquid suspending vehicle according to any preceding Claim, further comprising one or more of; buffer, flavouring, sweetener and/or preservative.

8. A liquid suspending vehicle according to Claim 7, wherein the buffer comprises one or more of citric acid, sodium citrate and/or sodium phosphate.

9. A liquid suspending vehicle according to Claim 7 or 8, wherein the sweetener content is less than 1% w/v and/or wherein the sweetener is one or more of sucralose, sorbitol, stevia, monk fruit sweetener or sucrose.

10. A liquid suspending vehicle according to any one of Claims 7, 8 or 9, wherein the preservative acts as an anti-microbial and/or anti-oxidising agent and/or wherein the preservative is one or more of potassium sorbate, sodium benzoate, methylparaben or propylparaben.

11. A liquid suspending vehicle according to any preceding Claim, further comprising one or more active ingredients which may be a medicament and wherein said medicament or active ingredient may be selected from one or more of; co-careldopa, sucralfate, spironolactone, clonazepam, solifenacin, diazepam, methyldopa, bisprolol, riboflavin or calcium carbonate.

12. A liquid suspending vehicle according to Claim 11, wherein the amount of active ingredient is in the range 2-21% w/v.

13. A liquid suspending vehicle according to any preceding Claim, wherein the formulation has a flow index of 0.46 and/or has a yield value in the range 10-60 dynes/cm².

14. A liquid suspending vehicle according to any preceding Claim, wherein the formulation is stable and does not degrade after one or more freeze-thaw cycle, say after one freeze-thaw cycle or after two or more freeze-thaw cycles.

15. A method for forming a liquid suspending vehicle of any preceding Claim, an exemplary method comprising;
i. Adding arrowroot starch and xanthan gum to water;
ii. Heating the mixture;
iii. Allowing or causing the mixture to cool, for example to room temperature;
iv. Adding a buffer solution to the cooled mixture of Step (iii) and mixing;
v. Adjusting the pH of the resulting formulation, as necessary, to the required pH.
